Europäisches Patentamt

European Patent Office

Office européen des brevets

(11) Publication number: **0 413 079 A2**

# EUROPEAN PATENT APPLICATION

(21) Application number: **90101643.6**

(22) Date of filing: **27.01.90**

(51) Int. Cl.⁵: **A61N 1/39, H02G 11/00**

(30) Priority: **15.08.89 JP 95704/89**

(43) Date of publication of application:
**20.02.91 Bulletin 91/08**

(84) Designated Contracting States:
**DE FR GB IT**

(71) Applicant: **FUKUDA DENSHI CO., LTD.**
**39-4, Hongo 3-chome Bunkyo-ku**
**Tokyo 113(JP)**

(72) Inventor: **Sakuma, Hisayoshi, c/o Funkuda Denshi Co., Ltd.**
**Shiroi enterprice place, 305-1, Nakaaza Nakadai**
**Shiroi-Cho, Inba-Gun, Saitama(JP)**
Inventor: **Ishihara, Isamu, c/o Funkuda Denshi Co., Ltd.**
**Shiroi enterprice place, 305-1, Nakaaza Nakadai**
**Shiroi-Cho, Inba-Gun, Saitama(JP)**

(74) Representative: **Behn, Klaus, Dipl.-Ing.**
**Patentanwalt Lindenberg 34**
**D-8134 Pöcking bei München(DE)**

(54) **Apparatus for treating a patient in an urgent case.**

(57) An apparatus for treating a patient in an urgent case, wherein one and another cords connect respectively one and another electrodes with an apparatus body.

The apparatus has one and another first cord hanging members being mounted on the one and another cords, respectively.

On the apparatus body, one and another second cord hanging members are mounted, and can engage with the first cord hanging members, respectively.

One and another cord hanging portions have the first cord hanging members and the second cord hanging members, respectively.

The cords can be hanged on the cord hanging portions along the apparatus body, and are removable from the cord hanging portions under pull force applied on themselves.

Fig. 2

## APPARATUS FOR TREATING A PATIENT IN AN URGENT CASE

BACKGROUND OF THE INVENTION

1. Field of the Invention

The present invention relates to an apparatus for treating a patient in an urgent case. More particularly, it relates to an apparatus for treating a patient in an apparatus urgent case, wherein a cord hanging portion is disposed, from which cord hanging portion a cord with on electrode can be removable directly in an urgent case.

2. Description of the Related Art

Generally, an apparatus for treating a patient in an urgent case is an apparatus which is used when it is necessary to treat a patient urgently, for example, as a defibrillator.

The urgent case is as follows.

That is to say, if the standstill and venticular fibrillation are neglected, the heart stops, which soon results in that a patient suffering from them dies, and therefore it is necessary that they should be treated urgently.

In such an urgent case, two electrodes, connected with an apparatus body by curled cords, are contacted closely to the breast of the patient, through which electrodes a high voltage pulse generated in the apparatus body is conducted into the living body of the patient.

Therefore, in order to cope with the urgent case, it is necessary that the electrodes are removed from the apparatus body as they are connected with cords, and that they are brought to the breast of the patient at once.

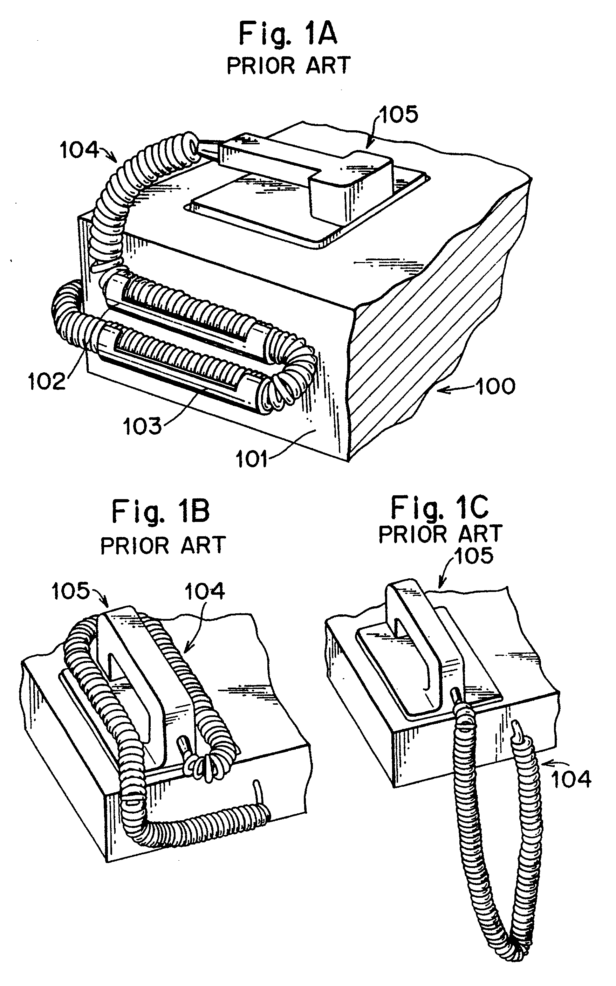

Figures 1A to 1C are explanatory drawings of the conventional apparatus for treating a patient in an urgent case.

Fig. 1A shows the conventional appratus wherein a cord hanging portion is disposed, and Figs. 1B and 1C show the conventional one wherein a cord hanging portion is not disposed.

With respect to the former, cord hanging portions 102 and 103 are integrated with an one side surface 101 of an apparatus body 100, and the same cord hanging portions are also integrated with another side surface of the apparatus body 100.

In an urgent case, it is necessary that an electrode 105 is removed from the apparatus body 100 at once, which electrode 105 is brought to the breast of the patient at 250cm's distance from the apparatus body 100 as the clectrode 105 is connected with a cord 104.

However, with respect to the conventional apparatus of Fig. 1A, when the electrode 105 is removed from the apparatus body 100, the cord 104 should be also removed from the cord hanging portions 102 and 103 at that time.

Thereby, the conventional apparatus of Fig. 1A has the defect that since it cannot cope with the urgent case, the efficiency of it's operation becomes down remarkably.

On the other hand, wite respect to the conventional apparatuses of Figs. 1B and 1C, when they are not used, a cord 104 is wound round an electrode 105 as shown in Fig. 1B, and a cord 104 is suspended down from an electrode 105 as shown in Fig. 1C.

However, regarding the conrential apparatus of Fig. 1B, since the cord 104 is wound round the electrode 105, there is the defect that the efficiency of it's operation becomes down remarkably as that of Fig. 1A. Regarding the conventional apparatus of Fig. 1C, since the cord 104 is suspended down, it may be snapped owing to the stress applied to itself, and thereby the apparatus cannot function as an urgent treating apparatus.

SUMMARY OF THE INVENTION

An object of the present invention is to elevate the efficiency of the operation of an urgent treating apparatus, and to display it's function so as to cope with an urgent case.

The above-mentioned object can be achieved by An apparatus for treating a patient in an urgent case, wherein one and another cords connect respectively one and another electrodes with an apparatus body, the apparatus comprising one and another first cord hanging members being mounted on said one and another cords,respectively,one and another second cord hanging members being mounted on said apparatus body, said second cord hanging members being capable of engaging with said first cord hanging members, respectively,one and another cord hanging portions having said first cord hanging members as well as said second cord hanging members, respectively, and said cords being capable of being hanged on said cord hanging portions, respectively, along said apparatus body, and being removable from said cord hanging portions, respectively, under pull force applied on themselves.

## BRIEF DESCRIPTION OF THE DRAWINGS

Further features and advantages of the present invention will be apparent from the ensuring dencription with reference to the accompanying drawings, wherein:

Fig. 1A to 1C are explamatory drawings of the conventional apparatus for treating a patient in an urgent case;

Fig. 2 is a whole drawing of an embodiment of the present invention;

Fig. 3A is a detailed drawing of the first embodiment of the present invention; and

Fig. 3B is a detailed drawing of the second embodiment of the present invention.

## DESCRIPTION OF THE PREFERRED EMBODIMENS

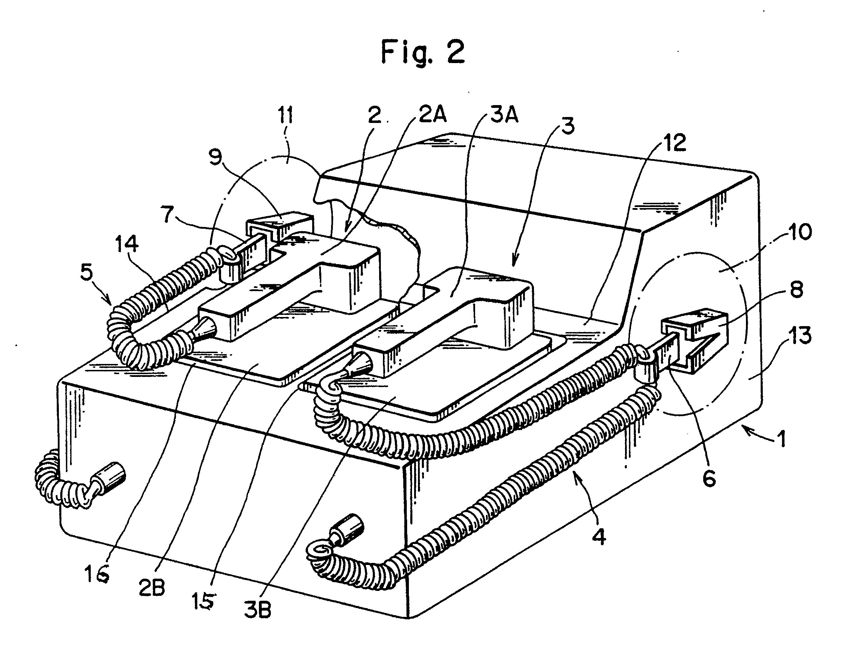

Figure 2 is a whole drawing of an embodiment of the present invention, wherein reference numeral 1 shows an apparatus body, 3 one electrode, 2 another electrode, 4 one cord, 5 another cord , 6 one first cord hanging member, 7 another first cord hanging member, 8 one second cord hanging member, 9 another second cord hanging member, 10 one cord hanging portion, and 11 another cord hanging portion.

The apparatus body 1 is made of plastics, for example, on the upper surface 12 of which apparatus body 1 the electrodes 2 and 3 are sit down in recesses 15 and 16, respectively.

The electrodes 2 and 3 consist of electrode plates 2B and 3B, as well as grips 2A and 3A, respectively, which electrodes 2 and 3 are connected with the appatus body 1 by the cords 4 and 5 projcting outerwards from the grips 2A and 3A.

The first cord hanging members 6 and 7 are mounted on the cords 4 and 5, respectively.

On the other hand, the second cord hanging members 8 and 9 are mounted on the apparatus body 1 so as to engage with the first cord hanging members 6 and 7, respectevely.

Concretely, the one second cord hanging member 8 is mounted at the rear area of the right side surface 13 of the apparatus body 1, and the another second cord hanging member 9 is mounted at the rear area of the left side surface 14 of the opparatus body 1.

The one cord hanging portion 10 is constituted by the one first and second cord hanging members 6 and 8, and the another cord hanging portion 11 is constituted by the another first and second cord hanging members 8 and 9.

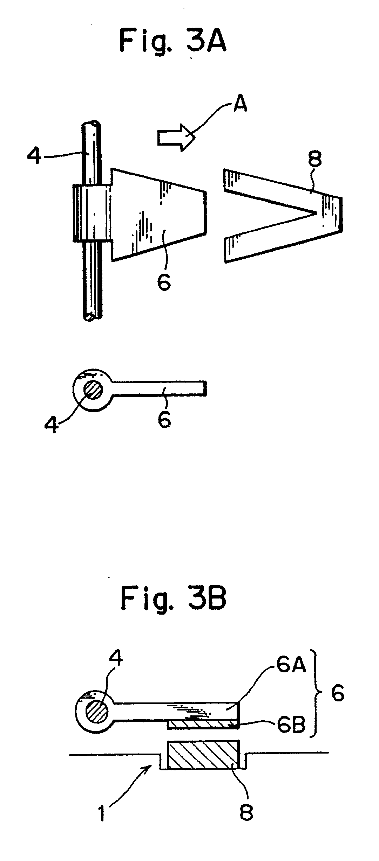

Figure 3A is a detailed drawing of the first embodiment of the present invention, and Figure 3B is a detailed drawing of the second embodiment of the present invention.

On referring to Figs. 3A and 3B, the one cord hanging partion 10 will be hereinafter only described, since the constitution of the another cord hanging portion 11 has the same constitution as the one cord hanging portion 10.

In the first embodiment (see Fig. 3A ), the first cord hanging member 6 is formed by a plate made of rubber, and the second cord hanging member 8 is formed by a holder in which the plate made of rubber may be accommodated.

The plate made of rubber, which constitutes the first cord hanging member 6, surrounds the cord 4 and bonds to it, as shown in the lower drawing of Fig. 3A.

On the other hand, the holder, which constitutes the second cord hanging member 6, is integrally formed with the apparatus body 1.

In the second embodiment ( see Fig. 3B ), the first cord hanging member 6 are formed by a plate 6A and an iron segment 6B anchored in the plate 6A, and the second cord hanging member 8 is formed by a magnet for absorbing the iron segment 6B which is anchored in the apparatus body 1 as shown in Fig. 3B.

The plate 6A, which constitutes the first cord hanging member 6, is formed by leather or cloth etc., and surrounds the cord 4 and bonds to it as the plate made of rubber of the first embodiment ( see Fig. 3A ).

Comparing the first embodiment ( see Fig. 3A ) with the second embodiment ( see Fig. 3B ) in view of elevating the efficiency of the operation in an urgent case, the second embodiment has more effect, since the cord 4 may be removable at once owing to use of the magnet .

The operation of the present invention will be hereinafter described.

When the apparatus for treating a patient in an urgent case is not used, the cords 4 and 5 are mounted on the cord hanging portions 10 and 11, resectively, along the apparatus body 1, by means of engaging the first cord hanging members 6 and 7 with the second cord hanging members 8 and 9, as shown in Fig. 2.

In such an urgent case as a patient becomes to suffer from standstill etc., the electrodes 2 and 3 are brought to the breast of the patient by taking the grips 2A and 3A with the hands of a doctor.

At this time, since pull forces are applied each to the cords 4 and 5 connected with the electrodes 2 and 3, the pull forces take off respectively the first cord hanging members 6 and 7 from the second cord hanging members 8 and 9.

That is to say, in an urgent case, the cords 4 and 5 may be removable at once from the cord hanging portions 10 and 11.

According to the present invention, the cord hanging portions 10 and 11 are constituted by two members, engaged each other, that is to say, the first cord hanging members 6 and 7 as well as the second cord hanging members 8 and 9.

Hence, in an urgent case, this two members may be separated each other, owing to the pull forces applied to the cords 4 and 5, as described hereinbefore.

Thereby, the cords 4 and 5 may be removable immediately from the apparatus body 1 in an urgent case, and accordingly, the efficiency of the operation, of the electrodes 3 and 2 connected with the cords 4 and 5, becomes to be elevated.

Moreover, the cords 4 and 5 are mounted on the cord hanging portions 10 and 11, being always along the apparatus body 1, as shown in Fig 2.

Hence, since the cords 4 and 5 are not suspended down ( see Fig. 1C ), they are not snapped.

Therefore, the apparatus can function as an urgent treating apparatus.

That is to say, the present invention has the effect that the efficiency of the operation becomes to be elevated and the function can be displayed so as to cope with an urgent case.

## Claims

1. An apparatus for treating a patient in an urgent case, wherein one and another cords connect respectively one and another electrodes with an apparatus body, the apparatus comprising; one and another first cord hanging members (6) and (7) being mounted on said one and another cords (4) and (5), respectively, one and another second cord hanging members (8) and (9) being mounted on said apparatus body (1), said second cord hanging members (8) and (9) being capable of engaging with said first cord hanging members (6) and (7), respectively, one and another cord hanging portions (10) and (11) having said first cord hanging members (6) and (7) aswell as said second cord hanging members (8) and (9), respectively, and said cords (4) and (5) being capable of being hanged on said cord hanging portions (10) and (11), respectively, along said apparatus body (1), and being removable from said cord hanging portions (10) and (11), respectively, under pull force applied on themselves.

2. An apparatus for treating a patient in an urgent case according to claim 1, wherein said cord hanging portions (10) and (11) are mounted respectively, at the rear areas of the right and left side surfaces (13) and (14) of said apparatus body (1).

3. An apparatus for treating a patient in an urgent case according to claim 1, wherein said first cord hanging members (6) and (7) are formed by plates made of rubber, each, and said second cord hanging members (8) and (9) are formed by holders in which said plates are accommodated, respectively.

4. An apparatus for treating a patient in an urgent case according to clim1, wherein said first cord hanging members (6) and (7) are composed of plates and iron segments anchored in said plates, each, and said cord hanging members (8) and (9) are formed by magnets being capable of absorbing said iron segments, respectively.

5. An apparatus for treating a patient in an urgent case according to claim 4, wherein said plates are formed by leatber or cloth.

## Fig. 1A
### PRIOR ART

## Fig. 1B
### PRIOR ART

## Fig. 1C
### PRIOR ART

Fig. 2

# Fig. 3A

# Fig. 3B